# EUROPEAN PATENT SPECIFICATION

(11) **EP 2 750 655 B1**
(45) Date of publication and mention of the grant of the patent: **21.02.2018**
(21) Application number: 12828981.6
(22) Date of filing: 29.08.2012
(51) Int. Cl.: A61K 8/29, A61K 8/26, A61K 8/30, A61Q 15/00, A61K 8/58, A61K 8/73, A61K 8/02, A61K 8/39, B29C 67/24

(54) **ANTIPERSPIRANT COMPOSITIONS AND PRODUCTS HAVING ENHANCED WETNESS PROTECTION AND METHODS FOR MAKING THE SAME**
SCHWEISSHEMMENDE ZUSAMMENSETZUNGEN UND PRODUKTE MIT ERHÖHTEM FEUCHTIGKEITSSCHUTZ SOWIE HERSTELLUNGSVERFAHREN DAFÜR
COMPOSITIONS ET PRODUITS ANTI-TRANSPIRATION AYANT UNE PROTECTION CONTRE L'HUMIDITÉ ACCRUE ET LEURS PROCÉDÉS DE FABRICATION

(30) Priority: 31.08.2011 US 201113222124
(43) Date of publication of application: 09.07.2014
(73) Proprietor: Henkel IP & Holding GmbH, 40589 Düsseldorf (DE)
(72) Inventor: GE, Haiyan, Scottsdale, Arizona 85254 (US); DOERING, Thomas, 41540 Dormagen (DE)
(74) Representative: Henkel IP Department
(86) International application number: PCT/US2012/052741
(87) International publication number: WO 2013/033129

(56) References cited:
- EP-A2- 0 135 315
- WO-A1-2009/023765
- US-A- 5 292 530
- US-A1- 2003 185 777
- US-A1- 2004 234 613
- US-A1- 2008 241 089
- US-A1- 2011 038 902
- US-A1- 2011 091 402
- WALTERS K A ET AL: "THE EFFECTS OF SURFACTANTS ON PENETRATION ACROSS THE SKIN", INTERNATIONAL JOURNAL OF COSMETIC SCIENCE, KLUWER ACADEMIC PUBLISHERS, DORDRECHT, NL, vol. 15, no. 6, 1 January 1993 (1993-01-01), pages 260-270, XP008036843, ISSN: 0142-5463

## Description

### FIELD OF THE INVENTION

The present invention relates generally to antiperspirant compositions, products, and methods for making the same, and more particularly relates to antiperspirant compositions that exhibit enhanced wetness protection, antiperspirant products comprising such antiperspirant compositions, and methods for making such antiperspirant compositions and products.

### BACKGROUND OF THE INVENTION

Antiperspirant and deodorant compositions are well known personal care products used to prevent or eliminate perspiration and body odor caused by perspiration. The compositions come in a variety of forms and may be formulated, for example, into aerosols, pumps, sprays, liquids, roll-ons, lotions, creams, sticks, and soft solids, etc.

There are various types of antiperspirant compositions that are desirable by a large majority of the population because of their ease of application and the presence of active antiperspirant compounds (e.g. antiperspirant salts) that prevent or block the secretion of perspiration and its accompanying odors. In one type, an antiperspirant salt is suspended in an anhydrous vehicle that often includes a solid water-insoluble wax. In a second type, an antiperspirant salt is dissolved in a liquid vehicle such as propylene glycol and gelled with a gelling agent such as dibenzylidene sorbitol. A third type includes an emulsion of an aqueous phase containing the antiperspirant salt and an oil phase containing, for example, a volatile silicone, fragrances, gellants, and other additives.

Stick antiperspirant products include an antiperspirant composition within a container. During use of the stick antiperspirant product, the top of the container is removed and the application surface of the composition is contacted with the skin, such as the underarm, by swiping or rubbing the stick across the skin. Sometimes the product also includes an undercap, or factory seal, that covers the application surface and is removed prior to first use. The container generally also includes some mechanism for moving the composition upwards through the container to continue to provide an exposed application surface.

WO 2009/023765A1 teaches the use of polyurethane urea powders as water or sweat absorbers in deodorants and antiperspirant compositions. Example 34 of this document discloses an antiperspirant gel powder stick comprising a) Aluminum Zirconium Tetrachlorohydrex Glycine as active antiperspirant compound and b) aluminum starch octenylsuccinate and isopropyl titanium triisostearate as moisture absorbent particles. The antiperspirant gel powder stick further comprises 2.75 wt. % of surfactants (Polysorbate 80 and hydrogenated castor oil). US 2003/0185777A1 discloses a water-free antiperspirant composition comprising a) at least two particulate polysaccharides, b) at least one astringent antiperspirant agent and c) at least one lipid component. The antiperspirant composition may contain emulsifiers/surfactants for further hydrophilization of the antiperspirant composition and, hence, optimized release of the antiperspirant agent and better removability of residues from the skin. US 2004/0234613A1, which is not concerned with antiperspirant compositions at all, is directed to coated powders wherein the coating comprises siloxy metal units and a process of providing a hybrid coating on a cosmetic particle. This document also discloses isopropyl titanium triisostearate coated particles, such as isopropyl titanium triisostearate coated TiO₂ or isopropyl titanium triisostearate coated red iron oxide. US 2011/0038902A1 discloses a method for manufacturing an antiperspirant product, the method comprising the steps of: depositing a first portion of the antiperspirant product into a mold, wherein the first portion comprises an emulsion having a water phase and an oil phase and wherein the oil phase comprises cetyl PEG/PPG-10/1 dimethicone; allowing the first portion to at least partially solidify; depositing a second portion of the antiperspirant product into the mold, wherein the second portion has a composition different from the first portion; and allowing the second portion to at least partially solidify. US 2008/0241089A1 discloses deodorant or antiperspirant stick composition in the form of an oil-in-water dispersion or emulsion, containing at least one lipid or wax component with a melting point >50° C, at least one specific nonionic oil-in-water emulsifier, at least one specific nonionic water-in-oil emulsifier, at least one specific oil, at least one watersoluble polyhydric C 2-C9 -alkanol with 2-6 hydroxyl groups and/or at least one watersoluble polyethylene glycol with 3-20 ethylene oxide units, 5% up to less than 50% by weight of water of the total composition, and at least one antiperspirant active substance. US 5292530A discloses an anhydrous composition, useful in topical cosmetic preparations, such as antiperspirants, that resists phase separation and exhibits improved properties comprising a topically-active compound, such as an astringent salt, an improved suspending agent comprising a finely-divided silica and a suspending wax composition; and a suitable volatile liquid carrier, such as a volatile silicone or a volatile hydrocarbon.

Commercial markets for antiperspirant and deodorant products are highly competitive, with consumers wanting products with increased antiperspirant efficacy for improved wetness protection. Efforts to increase the antiperspirant efficacy of solid wax antiperspirant products include incorporating super moisture absorbent materials, such as, for example, a polyacrylate homopolymer (sodium salt) and starch graft copolymers of poly(2-propenamide-co-2-propenioic acid) (sodium salt), into the antiperspirant composition together with one or more antiperspirant salts. By themselves, many of these super moisture absorbent materials can absorb a significant percentage of their weight in water. Unfortunately, when the super moisture absorbent materials are in a solid wax antiperspirant composition, any improvements in the antiperspirant efficacy are marginal at best because the presence of the antiperspirant salt typically produces an acidic environment that substantially reduces the water absorption performance of these super moisture absorbent materials. Moreover, the super moisture absorbent materials often become undesirably sticky or gummy in a solid wax antiperspirant composition when exposed to perspiration.

Accordingly, it is desirable to provide antiperspirant products that exhibit strong antiperspirant efficacy for wetness protection preferably without the antiperspirant composition becoming sticky or gummy when exposed to perspiration. Furthermore, other desirable features and characteristics of the present invention will become apparent from the subsequent detailed description of the invention and the appended claims, taken in conjunction with the accompanying drawings and this background of the invention.

### SUMMARY OF THE INVENTION

Antiperspirant compositions, products, and methods for making antiperspirant compositions and products are provided herein. A first subject-matter of this application is an antiperspirant composition comprising an active antiperspirant compound, moisture absorbent particles comprising aluminum starch octenylsuccinate and isopropyl titanium triisostearate, and a surfactant that comprises CH₃(CH₂)₁₇CH₂(OCH₂CH₂)₁₀OH, wherein the surfactant is present in an amount of from 0.05 to 1 wt. % of the antiperspirant composition.

Another subject-matter of this application is an antiperspirant product that comprises a container and an antiperspirant composition housed within the container, wherein the antiperspirant composition comprises an active antiperspirant compound, moisture absorbent particles comprising aluminum starch octenylsuccinate and isopropyl titanium triisostearate, and a surfactant that comprises CH₃(CH₂)₁₇CH₂(OCH₂CH₂)₁₀OH, wherein the surfactant is present in an amount of from 0.05 to 1 wt. % of the antiperspirant composition, a concentration effective to form micelles in the presence of moisture.

A third subject-matter of this application is a method for making an antiperspirant product, the method comprises the steps of mixing antiperspirant ingredients including an active antiperspirant compound together to form an antiperspirant premix; mixing moisture absorbent particles that comprise aluminum starch octenylsuccinate and isopropyl titanium triisostearate, a surfactant that comprises CH₃(CH₂)₁₇CH₂(OCH₂CH₂)₁₀OH, and a molten wax material together to form a molten wax-based premix; mixing the molten wax-based premix and the antiperspirant premix together to form an antiperspirant composition; depositing the antiperspirant composition is into a mold; and allowing the antiperspirant composition is to solidify, wherein the surfactant is present in an amount of from 0.05 to 1 wt. % of the antiperspirant composition.

### BRIEF DESCRIPTION OF THE DRAWINGS

Embodiments of the present invention will hereinafter be described in conjunction with the following drawing figures, wherein like numerals denote like elements, and wherein:
FIG. 1 is a perspective view of an antiperspirant product in accordance with an exemplary embodiment;
FIG. 2 is a top view of the antiperspirant product depicted in FIG. 1;
FIG. 3 is an exploded perspective view of an antiperspirant product in accordance with an exemplary embodiment; and
FIG. 4 is a flowchart of a method for making an antiperspirant product in accordance with an exemplary embodiment.

### DETAILED DESCRIPTION

The following Detailed Description is merely exemplary in nature and is not intended to limit the invention or the application and uses of the invention. Furthermore, there is no intention to be bound by any theory presented in the preceding Background of the Invention or the following Detailed Description.

The various embodiments contemplated herein relate to antiperspirant compositions that exhibit strong antiperspirant efficacy for enhanced wetness protection preferably without becoming sticky or gummy when exposed to perspiration, antiperspirant products comprising such antiperspirant compositions, and methods for making such antiperspirant compositions and products. Unlike the prior art, the exemplary embodiments taught herein form an antiperspirant composition that is a solid wax formulation and that comprises an active antiperspirant compound and moisture absorbent particles and a specific surfactant. The moisture absorbent particles comprise aluminum starch octenylsuccinate and isopropyl titanium triisostearate, and preferably can absorb up to at least about 22% of their weight in water when exposed to, for example, a 95% relative humidity (RH) or greater environment (e.g. perspiring skin of a user). Additionally, the inventors have found that the moisture absorbent particles can be regenerated during drier periods to absorb additional water later on for prolonged wetness protection. In particular, the moisture absorbent particles can release a significant portion of the initially absorbed water when subsequently exposed to a relatively low humidity environment of, for example, about 20% RH or less, and can reabsorb water if exposed again to a relatively high humidity environment. Moreover, the inventors have found that the moisture absorbent particles do not become undesirably sticky or gummy in a solid wax antiperspirant formulation when exposed to perspiration.

The antiperspirant composition further comprises a surfactant that comprises steareth-10, CH₃(CH₂)₁₇CH₂(OCH₂CH₂)₁₀OH, wherein the surfactant is present in an amount of from 0.05 to 1 wt. % of the antiperspirant composition. The surfactant is at or above its critical micelle concentration in the antiperspirant composition so that when the antiperspirant composition is exposed to perspiration, the surfactant molecules rearrange in the wax matrix to form micelles that attract and capture water and improve wetness protection. As used herein, the critical micelle concentration is defined as the concentration of the surfactant above which micelles are spontaneously formed in the antiperspirant composition in the presence of water. The inventors have surprisingly found that an antiperspirant composition comprising both the moisture absorbent particles and the surfactant has significantly improved water absorption performance both initially and throughout the day with less stickiness or gumminess than conventional antiperspirant compositions to provide continuous wetness protection preferably for a 12 hour period or longer. Without being limited by theory, it is believed that when the antiperspirant composition is spread across a user's skin and the user perspires, water diffuses through the wax matrix of the antiperspirant composition and the surfactant rearranges to form micelles. The micelles increase the osmotic pressure causing the water flux to increase across the wax matrix to draw in additional moisture from the perspiring skin. Initially, the active antiperspirant compound and the micelles preferentially absorb or capture water over the moisture absorption particles. The active antiperspirant compound, which continues to absorb water, diffuses into and swells up to plug the user's sweat glands to reduce further perspiring. With the active antiperspirant compound diffusing into the sweat glands, there is less active antiperspirant compound concentrated outside of the sweat glands and the moisture absorbent particles are able to absorb more moisture to further improve antiperspirant efficacy for enhanced and sustained wetness protection. Additionally, if the user stops perspiring, the moisture absorbent particles can regenerate to absorb additional water later on when needed to provide long lasting wetness protection.

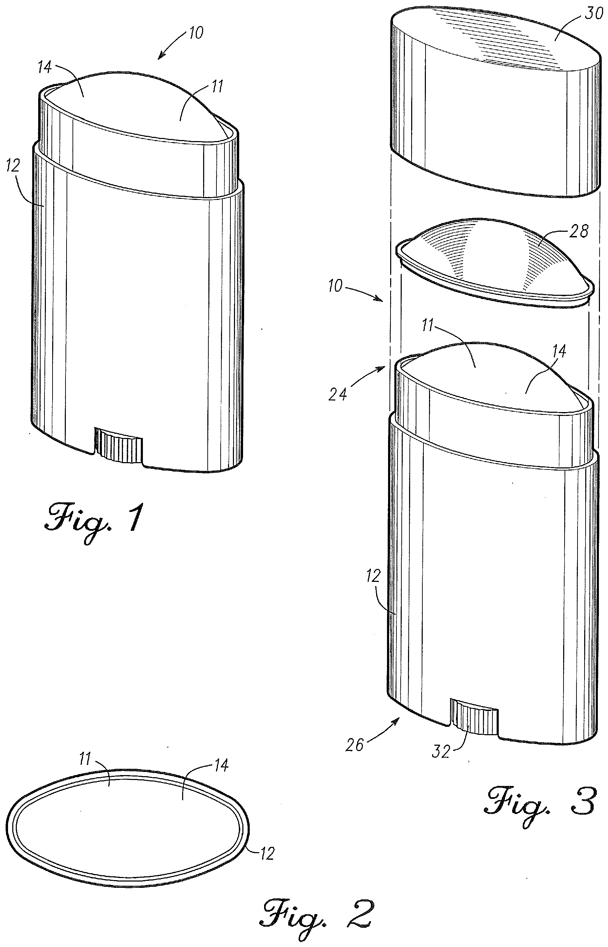

Referring to FIGS. 1 and 2, an antiperspirant product 10 in accordance with an exemplary embodiment is provided. The antiperspirant product 10 comprises an antiperspirant composition 11 that is preferably a solid wax formulation. As illustrated, the antiperspirant composition 11 has an application surface 14 that is substantially dome-shaped and that is configured to be applied to skin, such as, for example, an underarm. The antiperspirant product 10 may also comprise a container or dispenser 12 for dispensing the antiperspirant composition 11 to the skin.

The antiperspirant composition 11 contains at least one active ingredient (i.e. active antiperspirant compound), typically metal salts, that are thought to reduce perspiration by diffusing through the sweat ducts of eccrine glands and apocrine glands and hydrolyzing in the sweat ducts, where they combine with proteins to form an amorphous metal hydroxide agglomerate, plugging the sweat ducts so perspiration cannot diffuse to the skin surface. Some active antiperspirant compounds that may be used include astringent metallic salts, especially inorganic and organic salts of aluminum, zirconium, and zinc, as well as mixtures thereof. Particularly preferred are aluminum-containing and/or zirconium-containing salts or materials, such as aluminum halides, aluminum chlorohydrates, aluminum hydroxyhalides, zirconyl oxyhalides, zirconyl hydroxyhalides, and mixtures thereof. Exemplary aluminum salts include those having the general formula Al₂(OH)ₐCl_{b} x (H₂O), wherein a is from 2 to about 5; the sum of a and b is about 6; x is from about 1 to about 6; and wherein a, b, and x may have non-integer values. Exemplary zirconium salts include those having the general formula ZrO(OH)₂₋ₐClₐ x (H₂O), wherein a is from about 1.5 to about 1.87, x is from about 1 to about 7, and wherein a and x may both have non-integer values. Particularly preferred zirconium salts are those complexes that additionally contain aluminum and glycine, commonly known as ZAG complexes. These ZAG complexes contain aluminum chlorohydroxide and zironyl hyroxy chloride conforming to the above-described formulas. Examples of active antiperspirant compounds suitable for use in the various embodiments contemplated herein include aluminum dichlorohydrate, aluminum-zirconium octachlorohydrate, aluminum sesquichlorohydrate, aluminum chlorohydrex propylene glycol complex, aluminum dichlorohydrex propylene glycol complex, aluminum sesquichlorohydrex propylene glycol complex, aluminum chlorohydrex polyethylene glycol complex, aluminum dichlorohydrex polyethylene glycol complex, aluminum sesquichlorohydrex polyethylene glycol complex, aluminum-zirconium trichlorohydrate, aluminum zirconium tetrachlorohydrate, aluminum zirconium pentachlorohydrate, aluminum zirconium octachlorohydrate, aluminum zirconium trichlorohydrex glycine complex, aluminum zirconium tetrachlorohydrex glycine complex, aluminum zirconium pentachlorohydrex glycine complex, aluminum zirconium octachlorohydrex glycine complex, zirconium chlorohydrate, aluminum chloride, aluminum sulfate buffered, and the like, and mixtures thereof. In a preferred embodiment, the antiperspirant compound is aluminum zirconium trichlorohydrex glycine complex.

The active antiperspirant compound is preferably in a perspiration-reducing effective amount. In one embodiment, the antiperspirant composition 11 comprises an active antiperspirant compound present in the amount of from 14 to 25 wt. % (USP). As used herein, weight percent (USP) or wt. % (USP) of an antiperspirant salt is calculated as anhydrous weight percent in accordance with the U.S.P. method, as is known in the art. This calculation excludes any bound water and glycine.

The antiperspirant composition 11 contains moisture absorbent particles that comprise aluminum starch octenylsuccinate and isopropyl titanium triisostearate. In one example, the moisture absorbent particles are particles of aluminum starch octenylsuccinate surface treated with isopropyl titanium triisostearate. Preferably, the moisture absorbent particles have an average particle size of 20 µm or less, more preferably of from 1 to 20 µm, and most preferably of from 1 to 10 µm so that a user does not feel the moisture absorbent particles during application of the antiperspirant composition 11. In an exemplary embodiment, the aluminum starch octenylsuccinate is present in an amount of 96 to 99 weight percent (wt. %), and preferably of 98 wt. % of the moisture absorbent particles, and the isopropyl titanium triisostearate is present in an amount of 1 to 4 wt. %, and preferably of 2 wt. % of the moisture absorbent particles. The moisture absorbent particles are preferably present in an amount of 1 to 5 wt. %, more preferably 2 to 4 wt. %, and most preferably 3 wt. % of the antiperspirant composition 11. Moisture absorbent particles comprising aluminum starch octenylsuccinate and isopropyl titanium triisostearate are available from, for example, of Kobo Products, Inc. in South Plainfield, New Jersey under the trade name ASO-I2®.

The antiperspirant composition 11 further comprises in an amount of from 0.05 to 1 wt. % of the antiperspirant composition 11 a surfactant that comprises CH₃(CH₂)₁₇CH₂(OCH₂CH₂)₁₀OH, a waxy polyethylene glycol ether of stearic acid commonly referred to as steareth-10. The surfactant is present in the antiperspirant composition 11 at or above its critical micelle concentration. In one example, the critical micelle concentration for the surfactant in the antiperspirant composition 11 is 0.0018 mmol/l. Preferably the surfactant is present in an amount of from 0.2 to 0.8 wt. %, more preferably still from 0.4 to 0.6 wt. %, and most preferably 0.5 wt. % of the antiperspirant composition 11. Steareth-10 is available from, for example, Croda, Inc. in Edison, New Jersey under the trade name Brij® S10-SO. In one embodiment, a weight ratio of the moisture absorbent particles to the surfactant is from 1:1 to 6:1.

The antiperspirant composition 11 may comprise an anhydrous, hydrophobic vehicle, which includes a volatile silicone and/or a high melting component. In an exemplary embodiment, the active antiperspirant compound is suspended in the anhydrous, hydrophobic vehicle.

The high melting components may include any material suitable for use in an antiperspirant stick that melts at a temperature of about 70°C or higher. Typical of such materials are the high melting point waxes. These include beeswax, spermaceti, carnauba, bayberry, candelilla, montan, ozokerite, ceresin, paraffin waxes, semi-microcrystalline and microcrystalline waxes, hydrogenated jojoba oil, and hydrogenated castor oil (castor wax). The preferred wax is hydrogenated castor oil. Other suitable high melting components include various types of high melting gelling agents such as polyethylene-vinyl acetate copolymers, polyethylene homopolymers, 12-hydroxystearic acid, and substituted and unsubstituted dibenzylidene alditols. Typically, the high melting components comprise 1 to 25 wt. %, preferably 2 to 15 wt. %, of the composition. Volatile silicones include cyclomethicones and dimethicones, discussed above.

Other components may include, for example, non-volatile silicones, polyhydric alcohols having 3-6 carbon atoms and 2-6 hydroxy groups, fatty alcohols having from 12 to 24 carbon atoms, fatty alcohol esters, fatty acid esters, fatty amides, non-volatile paraffinic hydrocarbons, polyethylene glycols, polypropylene glycols, polyethylene and/or polypropylene glycol ethers of C₄-C₂₀ alcohols, polyethylene and/or polypropylene glycol esters of fatty acids, and mixtures thereof. The term "fatty" is intended to include hydrocarbon chains of 8 to 30 carbon atoms, preferably 12 to 18 carbon atoms.

Non-volatile silicones include polyalkylsiloxanes, polyalkylaryl siloxanes, and polyethersiloxanes with viscosities of 5 to 100,000 centistokes at 25°C, polymethylphenylsiloxanes with viscosities of 15 to 65 centistokes, and polyoxyalkylene ether dimethylsiloxane copolymers with viscosities of 1200 to 1500 centistokes.

Useful polyhydric alcohols include propylene glycol, butylenes glycol, dipropylene glycol and hexylene glycol. Fatty alcohols include stearyl alcohol, cetyl alcohol, myristyl alcohol, oleyl alcohol, and lauryl alcohol. Fatty alcohol esters include C₁₂₋₁₅ alcohols benzoate, myristyl lactate, cetyl acetate, and myristyl octanoate. Fatty acid esters include isopropyl palmitate, myristyl myristate, and glyceryl monostearate. Fatty amides include stearamide MEA, stearamide MEA-stearate, lauramide DEA, and myristamide MIPA. In an exemplary embodiment, the antiperspirant composition 11 comprises stearyl alcohol in an amount of from 18 to 22 wt. % of the antiperspirant composition 11. In particular, the inventors have found that the presence of stearyl alcohol in the antiperspirant composition 11 facilitates the surfactant rearranging to micelles due to the similarities in chemical structures of the surfactant (e.g. CH₃(CH₂)₁₇CH₂(OCH₂CH₂)₁₀OH) and stearyl alcohol (e.g. CH₃(CH₂)₁₇OH).

Non-volatile paraffinic hydrocarbons include mineral oils and branched chain hydrocarbons with 16 to 68, preferably 20 to 40, carbon atoms. Suitable polyethylene glycols and polypropylene glycols will typically have molecular weights of 500 to 6000, such as PEG-10, PEG-40, PEG-150 and PPG-20, often added as rheology modifiers to alter product appearance or sensory attributes.

Polyethylene and/or polypropylene glycol ethers or C₄-C₂₀ alcohols include PPG-10 butanediol, PPG-14 butyl ether, PPG-5-buteth-7, PPG-3-isostearth-9, PPG-3-myreth-3, oleth-10, and steareth-20. Polyethylene and/or polypropylene glycol esters of fatty acids include PEG-8 distearate, PEG-10 dioleate, and PPG-26 oleate. These are generally added to give emollient properties.

The antiperspirant composition 11 contemplated herein also may comprise additives, such as those used in conventional antiperspirants. For example, in addition to antiperspirant efficacy, the antiperspirant composition 11 may comprise additives that cause the antiperspirant composition 11 to exhibit long-lasting fragrance, odor protection, bacteria control, and/or another desired purpose and/or function. These additives include, but are not limited to, fragrances, including encapsulated fragrances, dyes, pigments, preservatives, antioxidants, moisturizers, and the like. These optional ingredients can be included in the antiperspirant composition 11 in an amount of 0 to 20 wt. %.

The above list of materials is by way of example only and is not intended to be a comprehensive list of all potential components of the antiperspirant products contemplated herein. Other high and low melting waxes, volatile and non-volatile compounds and other suitable components are readily identifiable to those skilled in the art. Of course, other ingredients such as colloidal silica, fumed silica, particulate polyolefins, talcum materials, colorants and preservatives may also be included as desired. For example, the antiperspirant composition 11 may include up to 5% fragrance or 2% colorant by weight.

As noted above, in addition to an active antiperspirant compound, the antiperspirant composition 11 may comprise a component or components that cause it to exhibit or impart a desired function or purpose in addition to antiperspirant efficacy. For example, the antiperspirant composition 11 may comprise deodorant active ingredients. A suitable deodorant active ingredient is any agent that inhibits, suppresses, masks or neutralizes malodor. These may include (1) antimicrobial or bactericidal agents that kill the bacteria responsible for malodor production, (2) agents that inhibit or suppress or interfere with the bacterial enzymatic pathway that produces malodor, and (3) agents that mask or absorb or neutralize malodor. "Fragrances" as used herein are not considered deodorant active ingredients. Examples of deodorant actives ingredients include triclosan, triclocarban, usnic acid salts, zinc phenolsulfonate, b-chloro-D-alanine, D-cycloserine, animooxyacetic acid, cyclodextrine, and sodium bicarbonate. Alternatively, or in addition, the antiperspirant composition 11 may comprise fragrances, for example, in an amount that imparts a long-lasting fragrance to the antiperspirant composition 11.

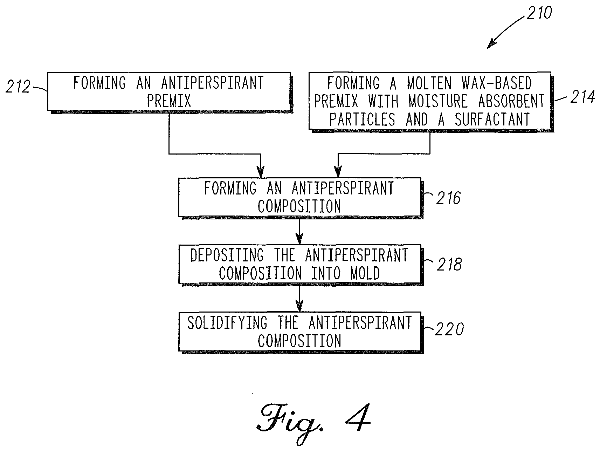

In accordance with exemplary embodiments, a method 210 for making the antiperspirant product 10 illustrated in FIGS. 1 and 2 is shown in FIGS. 3-4. With reference to FIG. 4, the method 210 comprises mixing antiperspirant ingredients including an active antiperspirant compound together to form an antiperspirant premix (step 212). In one example, cyclopentasiloxane is agitated in a mixing container. Fumed silica (e.g. Aerosil R872) and silica (Aerosil 300) are incrementally added to the mixing container and agitated until all of the silica is wetted with the cyclopentasiloxane. An active antiperspirant compound (e.g. aluminum zirconium trichlorohydrex-GLY) is then slowly added to the mixing container and is mixed until the blend appears homogeneous. High shear mixing is then used to mix the blend until the appearance is consistently fluid and absent of particulates to form the antiperspirant premix.

Moisture absorbent particles (e.g. aluminum starch octenylsuccinate and isopropyl titanium triisostearate), a surfactant (e.g. steareth-10), and a molten wax material are mixed together to form a molten wax-based premix (step 214). In one example, waxes including castor wax and stearyl alcohol (e.g. alcohol-based wax) are combined with PPG-14 butyl ether and are heated to a temperature of 85°C or less and slowly agitated to form a molten wax mixture. A surfactant (e.g. Brij® S10-SO) is incrementally added and agitated until it melts and becomes homogeneous with the molten wax mixture. Moisture absorbent particles (e.g. ASO-I2®) are then added and agitated until they become homogeneous with the molten wax mixture and the surfactant to form the molten wax-based premix.

The molten wax-based premix and the antiperspirant premix are mixed together to form the antiperspirant composition (step 216) as discussed in the foregoing paragraphs. In one example, the antiperspirant premix is added to the molten wax-based premix and mixed at a temperature of from 64 to 69°C until the mixture is homogeneous. Cyclopentasiloxane, which is at room temperature, is then added to the mixture and the mixture is maintained at a temperature of 60°C and mixed until it is homogeneous to form the antiperspirant composition.

Referring to FIGS. 3-4, the antiperspirant product is deposited in molten form into a mold (step 218) and solidified (step 220). The molten temperature of the antiperspirant product is generally in the range of from 50 to 85°C. The antiperspirant product is cooled below 50°C, to produce the antiperspirant product in a solid form. The container 12 may be used as the mold for the antiperspirant product to form the antiperspirant composition 11 illustrated in FIG. 3. It will be appreciated, however, that the invention is not limited to use of the container as a mold and that any satisfactory mold may be used for manufacturing the antiperspirant product.

In an exemplary embodiment, the container 12 has an application end 24 and an opposite end 26. The container 12 also contains a factory seal 28, which is positioned over the application surface 14 of antiperspirant composition 11 to protect it during shipment and to render it tamper-proof prior to purchase, and a cover 30. The factory seal 28 is removed by the user, and the cover is used during storage of the product between uses. As the product is exhausted, it is advanced from the container 12 by the user using advancement device 32, e.g., a screw mechanism as shown, at opposite end 26 of container 12.

As discussed in U.S. Patent Number 5,753,212 filed September 16, 1996, the antiperspirant composition 11 may be molded into the container 12 by first sealing the application end 24 of the container 12 with the factory seal 28 and introducing a predetermined quantity of molten antiperspirant composition 11 through the open opposite end 26. The antiperspirant composition 11 is then cooled to its non-molten form for example, by passing the filled container through a forced air tunnel operating at between 10 to 25°C. The finished product (FIG. 3) is completed by sealing the open opposite end 26 with a package base (not shown) that includes the advancement device 32. Other suitable methods for molding and/or forming the antiperspirant product known to those skilled in the art may also be used.

The following is an example of the antiperspirant product in accordance with an exemplary embodiment. The example is provided for illustration purposes only and is not meant to limit the various embodiments of the antiperspirant product in any way. All materials are set forth in weight percent.

### EXAMPLE - ANTIPERSPIRANT COMPOSITION

Antiperspirant Product - Solid Wax Formulation

| Ingredient | Wt. % |
|---|---|
| Castor Wax | 2.5 to 3 |
| Stearyl Alcohol | 18 to 22 |
| PPG-14 Butyl Ether | 9 to 11 |
| Steareth-10 | 0.05 to 1 |
| Aluminum Starch Octenylsuccinate and Isopropyl Titanium Triisostearate | 1 to 5 |
| Aluminum Zironium Trichlorohydrex-Gly | 18 to 25 |
| Cyclopentasiloxane | 35 to 45 |
| Fumed Silica (Aerosil R972 V) | 1.25 to 1.5 |
| Silica (Aerosil 300) | 0.32 to 0.375 |
| Total | 100.0 |

Accordingly, antiperspirant products compositions that exhibit strong antiperspirant efficacy for enhanced wetness protection preferably without becoming sticky or gummy when exposed to perspiration, antiperspirant products comprising such antiperspirant compositions, and methods for making such antiperspirant compositions and products have been described.

While at least one exemplary embodiment has been presented in the foregoing Detailed Description, it should be appreciated that a vast number of variations exist. It should also be appreciated that the exemplary embodiment or exemplary embodiments are only examples, and are not intended to limit the scope, applicability, or configuration of the invention in any way. Rather, the foregoing Detailed Description will provide those skilled in the art with a convenient road map for implementing an exemplary embodiment of the invention, it being understood that various changes may be made in the function and arrangement of elements described in an exemplary embodiment without departing from the scope of the invention as set forth in the appended Claims.

## Claims

1. An antiperspirant composition comprising:
an active antiperspirant compound;
moisture absorbent particles comprising aluminum starch octenylsuccinate and isopropyl titanium triisostearate; and
a surfactant that comprises CH₃(CH₂)₁₇CH₂(OCH₂CH₂)₁₀OH, wherein the surfactant is present in an amount of from 0.05 to 1 wt. % of the antiperspirant composition.

2. The antiperspirant composition according to claim 1, wherein the moisture absorbent particles comprise aluminum starch octenylsuccinate that is surface treated with isopropyl titanium triisostearate.

3. The antiperspirant composition according to claim 1, wherein the moisture absorbent particles are present in an amount of from 1 to 5 wt. %, preferably of from 2 to 4 wt. % the antiperspirant composition.

4. The antiperspirant composition according to claim 1, wherein the surfactant is present in an amount of from 0.2 to 0.8 wt. % of the antiperspirant composition.

5. The antiperspirant composition according to claim 1, wherein a weight ratio of the moisture absorbent particles to the surfactant is from 1:1 to 6:1.

6. An antiperspirant product comprising:
a container; and
an antiperspirant composition housed within the container and comprising:
an active antiperspirant compound;
moisture absorbent particles comprising aluminum starch octenylsuccinate and isopropyl titanium triisostearate; and
a surfactant comprising CH₃(CH₂)₁₇CH₂(OCH₂CH₂)₁₀OH wherein CH₃(CH₂)₁₇CH₂(OCH₂CH₂)₁₀OH is present in an amount of from 0.05 to 1 wt. % of the antiperspirant composition.

7. The antiperspirant product according to claim 6, wherein the concentration of CH₃(CH₂)₁₇CH₂(OCH₂CH₂)₁₀OH in the antiperspirant composition is at least 0.0018 mmol/l.

8. The antiperspirant product according to claim 6, wherein aluminum starch octenylsuccinate is present in an amount of 96 to 99 wt. % of the moisture absorbent particles, and isopropyl titanium triisostearate is present in an amount of 1 to 4 wt. % of the moisture absorbent particles.

9. The antiperspirant product according to claim 6, wherein the moisture absorbent particles are present in an amount of from 1 to 5 wt. % of the antiperspirant composition.

10. The antiperspirant product according to claim 6, wherein the antiperspirant composition is a solid wax formulation that comprises stearyl alcohol that is present in an amount of 18 to 22 wt. % of the antiperspirant composition.

11. The antiperspirant product according to claim 6, wherein the moisture absorbent particles are present in an amount of from 2 to 4 wt. % of the antiperspirant composition.

12. The antiperspirant product according to claim 6, wherein CH₃(CH₂)₁₇CH₂(OCH₂CH₂)₁₀OH is present in an amount of from 0.2 to 0.8 wt. % of the antiperspirant composition.

13. A method for making an antiperspirant product, the method comprising the steps of:
mixing antiperspirant ingredients including an active antiperspirant compound together to form an antiperspirant premix;
mixing moisture absorbent particles that comprise aluminum starch octenylsuccinate and isopropyl titanium triisostearate, a surfactant that comprises CH₃(CH₂)₁₇CH₂(OCH₂CH₂)₁₀OH, and a molten wax material together to form a molten wax-based premix;
mixing the molten wax-based premix and the antiperspirant premix together to form an antiperspirant composition;
depositing the antiperspirant composition into a mold; and
allowing the antiperspirant composition to solidify,
wherein the surfactant is present in an amount of from 0.05 to 1 wt. % of the antiperspirant composition.

## Patentansprüche

1. Schweißhemmende Zusammensetzung, umfassend:
eine aktive schweißhemmende Verbindung;
feuchtigkeitsabsorbierende Partikel, umfassend Aluminiumstärkeoctenylsuccinat und Isopropyltitantriisostearat; und
ein Tensid, das CH₃(CH₂)₁₇CH₂(OCH₂CH₂)₁₀OH umfasst, wobei das Tensid in einer Menge von 0,05 bis 1 Gew.-% der schweißhemmenden Zusammensetzung vorliegt.

2. Schweißhemmende Zusammensetzung nach Anspruch 1, wobei die feuchtigkeitsabsorbierenden Partikel Aluminiumstärkeoctenylsuccinat umfassen, das mit Isopropyltitantriisostearat oberflächenbehandelt ist.

3. Schweißhemmende Zusammensetzung nach Anspruch 1, wobei die feuchtigkeitsabsorbierenden Partikel in einer Menge von 1 bis 5 Gew.-%, vorzugsweise von 2 bis 4 Gew.-% der schweißhemmenden Zusammensetzung vorliegen.

4. Schweißhemmende Zusammensetzung nach Anspruch 1, wobei das Tensid in einer Menge von 0,2 bis 0,8 Gew.-% der schweißhemmenden Zusammensetzung vorliegt.

5. Schweißhemmende Zusammensetzung nach Anspruch 1, wobei ein Gewichtsverhältnis von den feuchtigkeitsabsorbierenden Partikeln zu dem Tensid 1:1 bis 6:1 beträgt.

6. Schweißhemmendes Produkt, umfassend:
einen Behälter; und
eine schweißhemmende Zusammensetzung, die in dem Behälter aufgenommen ist und umfasst:
eine aktive schweißhemmende Verbindung;
feuchtigkeitsabsorbierende Partikel, umfassend Aluminiumstärkeoctenylsuccinat und Isopropyltitantriisostearat; und
ein Tensid, umfassend CH₃(CH₂)₁₇CH₂(OCH₂CH₂)₁₀OH, wobei CH₃(CH₂)₁₇CH₂(OCH₂CH₂)₁₀OH in einer Menge von 0,05 bis 1 Gew.-% der schweißhemmenden Zusammensetzung vorliegt.

7. Schweißhemmendes Produkt nach Anspruch 6, wobei die Konzentration von CH₃(CH₂)₁₇CH₂(OCH₂CH₂)₁₀OH in der schweißhemmenden Zusammensetzung mindestens 0,0018 mmol/l beträgt.

8. Schweißhemmendes Produkt nach Anspruch 6, wobei Aluminiumstärkeoctenylsuccinat in einer Menge von 96 bis 99 Gew.-% der feuchtigkeitsabsorbierenden Partikel vorliegt und Isopropyltitantriisostearat in einer Menge von 1 bis 4 Gew.-% der feuchtigkeitsabsorbierenden Partikel vorliegt.

9. Schweißhemmendes Produkt nach Anspruch 6, wobei die feuchtigkeitsabsorbierenden Partikel in einer Menge von 1 bis 5 Gew.-% der schweißhemmenden Zusammensetzung vorliegen.

10. Schweißhemmendes Produkt nach Anspruch 6, wobei die schweißhemmende Zusammensetzung eine feste Wachsformulierung ist, die Stearylalkohol, der in einer Menge von 18 bis 22 Gew.-% der schweißhemmenden Zusammensetzung vorliegt, umfasst.

11. Schweißhemmendes Produkt nach Anspruch 6, wobei die feuchtigkeitsabsorbierenden Partikel in einer Menge von 2 bis 4 Gew.-% der schweißhemmenden Zusammensetzung vorliegen.

12. Schweißhemmendes Produkt nach Anspruch 6, wobei CH₃(CH₂)₁₇CH₂(OCH₂CH₂)₁₀OH in einer Menge von 0,2 bis 0,8 Gew.-% der schweißhemmenden Zusammensetzung vorliegt.

13. Verfahren zum Herstellen eines schweißhemmenden Produkts, wobei das Verfahren die Schritte umfasst:
Mischen schweißhemmender Inhaltsstoffe, einschließlich einer aktiven schweißhemmenden Verbindung, miteinander, um ein schweißhemmendes Vorgemisch auszubilden;
Mischen feuchtigkeitsabsorbierender Partikel, die Aluminiumstärkeoctenylsuccinat und Isopropyltitantriisostearat umfassen, eines Tensids, das CH₃(CH₂)₁₇CH₂(OCH₂CH₂)₁₀OH umfasst, und eines geschmolzenen Wachsmaterials miteinander, um ein Vorgemisch auf Basis eines geschmolzenen Wachses auszubilden;
Mischen des Vorgemischs auf Basis des geschmolzenen Wachses und des schweißhemmenden Vorgemischs miteinander, um eine schweißhemmende Zusammensetzung auszubilden;
Abscheiden der schweißhemmenden Zusammensetzung in eine Form; und
Ermöglichen, dass sich die schweißhemmende Zusammensetzung verfestigt,
wobei das Tensid in einer Menge von 0,05 bis 1 Gew.-% der schweißhemmenden Zusammensetzung vorliegt.

## Revendications

1. Composition antisudorifique comprenant :
un composé actif antisudorifique ;
des particules absorbant l'humidité comprenant de l'octénylsuccinate d'amidon et d'aluminium et du triisostéarate de titane et d'isopropyle ; et
un tensioactif qui comprend du CH₃(CH₂)₁₇CH₂(OCH₂CH₂)₁₀OH, le tensioactif étant présent dans une quantité comprise entre 0,05 et 1 % en poids de la composition antisudorifique.

2. Composition antisudorifique selon la revendication 1, dans laquelle les particules absorbant l'humidité comprennent de l'octénylsuccinate d'amidon et d'aluminium qui est traité en surface avec du triisostéarate de titane et d'isopropyle.

3. Composition antisudorifique selon la revendication 1, dans laquelle les particules absorbant l'humidité sont présentes dans une quantité comprise entre 1 et 5 % en poids, de préférence entre 2 et 4 % en poids de la composition antisudorifique.

4. Composition antisudorifique selon la revendication 1, dans laquelle le tensioactif est présent dans une quantité comprise entre 0,2 et 0,8 % en poids de la composition antisudorifique.

5. Composition antisudorifique selon la revendication 1, dans laquelle un rapport pondéral entre les particules absorbant l'humidité et le tensioactif est compris entre 1:1 et 6:1.

6. Produit antisudorifique comprenant :
un récipient ; et
une composition antisudorifique contenue dans le récipient et comprenant :
un composé actif antisudorifique ;
des particules absorbant l'humidité comprenant de l'octénylsuccinate d'amidon et d'aluminium et du triisostéarate de titane et d'isopropyle ; et
un tensioactif comprenant du CH₃(CH₂)₁₇CH₂(OCH₂CH₂)₁₀OH, le CH₃(CH₂)₁₇CH₂(OCH₂CH₂)₁₀OH étant présent dans une quantité comprise entre 0,05 et 1 % en poids de la composition antisudorifique.

7. Produit antisudorifique selon la revendication 6, dans lequel la concentration de CH₃(CH₂)₁₇CH₂(OCH₂CH₂)₁₀OH dans la composition antisudorifique est au moins de 0,0018 mmol/l.

8. Produit antisudorifique selon la revendication 6, dans lequel l'octénylsuccinate d'amidon et d'aluminium est présent dans une quantité comprise entre 96 et 99 % en poids des particules absorbant l'humidité, et le triisostéarate de titane et d'isopropyle est présent dans une quantité comprise entre et 1 et 4 % en poids des particules absorbant l'humidité.

9. Produit antisudorifique selon la revendication 6, dans lequel les particules absorbant l'humidité sont présentes dans une quantité comprise entre 1 et 5 % en poids de la composition antisudorifique.

10. Produit antisudorifique selon la revendication 6, dans lequel la composition antisudorifique est une formulation de cire solide qui comprend de l'alcool stéarylique qui est présent dans une quantité comprise entre 18 et 22 % en poids de la composition antisudorifique.

11. Produit antisudorifique selon la revendication 6, dans lequel les particules absorbant l'humidité sont présentes dans une quantité comprise entre 2 et 4 % en poids de la composition antisudorifique.

12. Produit antisudorifique selon la revendication 6, dans lequel le CH₃(CH₂)₁₇CH₂(OCH₂CH₂)₁₀OH est présent dans une quantité comprise entre 0,2 et 0,8 % en poids de la composition antisudorifique.

13. Procédé de fabrication d'un produit antisudorifique, le procédé comprenant les étapes suivantes :
mélanger ensemble des ingrédients antisudorifiques, dont un composé antisudorifique actif, pour former un prémélange antisudorifique ;
mélanger ensemble des particules absorbant l'humidité qui comprennent de l'octénylsuccinate d'amidon et d'aluminium et du triisostéarate de titane et d'isopropyle, un tensioactif qui comprend du CH₃(CH₂)₁₇CH₂(OCH₂CH₂)₁₀OH et une matière de cire fondue pour former un prémélange à base de cire fondue ;
mélanger ensemble le prémélange à base de cire fondue et le prémélange antisudorifique pour former une composition antisudorifique ;
déposer la composition antisudorifique dans un moule ; et
laisser la composition antisudorifique se solidifier,
le tensioactif étant présent dans une quantité comprise entre 0,05 et 1 % en poids de la composition antisudorifique.
